# FASCICULE DE BREVET EUROPEEN

(11) **EP 1 242 404 B1**
(45) Date de publication et mention de la délivrance du brevet: **07.07.2004**
(21) Numéro de dépôt: 00988936.1
(22) Date de dépôt: 15.12.2000
(51) Int. Cl.: C07D 401/04, A61K 31/4545, A61K 31/4523, A61P 1/00, C07D 401/14, C07D 211/58

(54) **PHENOXYPROPANOLAMINES, LEUR PREPARATION ET LEUR APPLICATION EN THERAPEUTIQUE**
PHENOXYPROPANOLAMIN-DERIVATE, IHRE HERSTELLUNG UND THERAPEUTISCHE VERWENDUNG
PHENOXYPROPANOLAMINES, PREPARATION AND THERAPEUTIC USE THEREOF

(30) Priorité: 17.12.1999 FR 9915933
(43) Date de publication de la demande: 25.09.2002
(73) Titulaire: SANOFI-SYNTHELABO, 75013 Paris (FR)
(72) Inventeur: BOVY, Philippe, R., F-78750 Mareil Marly (FR); CECCHI, Roberto, I-26900 Lodi (IT); VENIER, Olivier, F-94160 Saint Mandé (FR)
(74) Mandataire: Varady, Peter
(86) Numéro de dépôt international: PCT/FR2000/003559
(87) Numéro de publication internationale: WO 2001/044227

(56) Documents cités:
- EP-A- 0 095 454
- WO-A-99/65895
- DE-A- 3 524 955
- US-A- 5 627 196
- M HORI ET AL: "A soluble polymer approach to the fishing out principle: synthesis and purification of beta-amino alcohols" JOURNAL OF ORGANIC CHEMISTRY,US,AMERICAN CHEMICAL SOCIETY. EASTON, vol. 63, no. 3, 6 février 1998 (1998-02-06), pages 889-894-894, XP002110346 ISSN: 0022-3263 cité dans la demande

## Description

La présente invention concerne de nouvelles phénoxypropanolamines, les compositions pharmaceutiques les contenant, un procédé pour leur préparation et des intermédiaires dans ce procédé.

BE902897 décrit des aryloxypropanolamines portant un groupe 4-pipéridininyl-1-substitué sur l'amine, ces composés ayant une activité β₁-bloquante et α-bloquante.

J. Org. Chem., 1988, 63:889 :894, décrit d'autres aryloxypropanolamines portant un groupe 4-pipéridinyl-1-substitué sur l'amine.

Il a été maintenant trouvé que des phénoxypropanolamines portant un radical 1-(pyrid-2-yl)-pipéridin-4-yl sur l'amine possèdent une activité agoniste vis-à-vis des récepteurs β3-adrénergiques.

Ainsi, la présente invention concerne, selon un de ses aspects, des phénoxypropanolamines de formule (I) 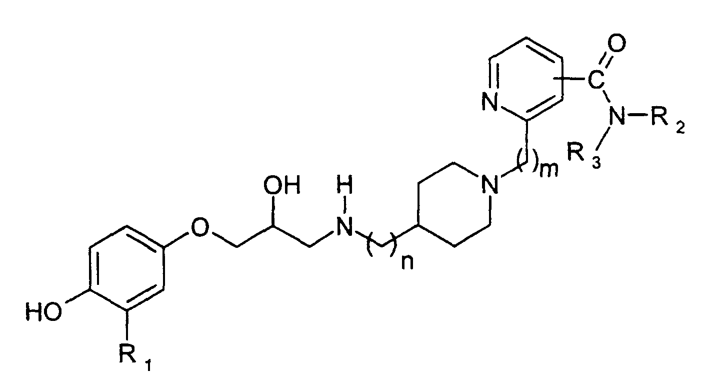 dans laquelle
R₁ représente un hydrogène, un groupe -S(O)_{z}-(C₁-C₄)Alk-R',
(R' étant H, phényle ou (C₁-C₄)alcoxy ),-NHSO₂-(C₁-C₄)Alk, NHCO(C₁-C₄)Alk ;
m et n sont chacun indépendamment 0, 1 ou 2 ;
R₂ et R₃ représentent indépendamment un hydrogène, un groupe (C₅-C₆)alkyle, (C₃-C₆)cycloalkyle, (C₁-C₄)alcoxy, hydroxy(C₁-C₄)alkyle, (C₁-C₄)alcoxy-(C₁-C₄)alkyle, mono- ou di-(C₁-C₄)Alkamino(C₁-C₄)alkyle, pyrrolidino(C₁-C₄) alkyle, phénylamino, (C₂-C₄)alkenyle, un groupe aromatique ou hétéroaromatique substitué ou non par un groupe R₄, un groupe aralkyle ou hétéroaralkyle substitué ou non par un groupe R₄ ; R₂ et R₃ ne pouvant représenter simultanément l'hydrogène ;
R₂ et R₃ peuvent aussi ensemble constituer un cycle saturé ou insaturé de 3 à 8 atomes pouvant porter un substituant (C₁-C₄)alkyle, amino(C₁-C₄) alkyle, carbamoyle ou benzyle, ;
R₄ représente un hydrogène ou un halogène, un groupe -CO(C₁-C₄)Alk ou un groupe -NHSO₂-(C₁-C₄)Alk ; un groupe (C₁-C₄)Alk, un groupe (C₁-C₄)alkoxy, -COOH, -COO(C₁-C₄)Alk, -CN, -CONR₃R₄, -NO₂, -SO₂NH₂, -NHSO₂(C₁-C₄)Alk ;
z est 1 ou 2 ;
et leurs sels ou solvates.

Dans la présente description le terme « (C₁-C₄)Alk » désigne un radical monovalent d'un hydrocarbure en C₁-C₄ saturé à chaîne droite ou ramifiée. Dans la présente description le terme « aralkyle » désigne un radical divalent alkyle saturé à chaîne droite ou ramifiée porteur d'un noyau aromatique. Dans la présente description le terme « hétéroaralkyle » désigne un radical divalent alkyle saturé à chaîne droite ou ramifiée porteur d'un noyau hétéroaromatique.

Les sels des composés de formule (I) et (Ia) selon la présente invention comprennent aussi bien les sels d'addition avec des acides minéraux ou organiques pharmaceutiquement acceptables tels que le chlorhydrate, le bromhydate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I) ou (Ia) tels que le picrate, l'oxalate ou les sels d'addition avec des acides optiquement actifs, par exemple les acides camphosulfoniques et les acides mandéliques ou mandéliques substitués.

De plus, lorsque les composés de formule (I) possèdent un groupe carboxy libre les sels comprennent aussi les sels avec des bases minérales, de préférence celles avec des métaux alcalins tels que le sodium ou le potassium, ou avec des bases organiques.

Les stéréoisomères optiquement purs, ainsi que les mélanges d'isomères des composés de formule (I), dus aux carbones asymétriques ou au groupe sulfinyle dans la signification de R₁, dans une proportion quelconque, sont partie de la présente invention.

Des composés préférés de la présente invention comprennent les composés de formule (I) où le groupe CONR₂R₃ est dans la position 5 de la pyridine.

D'autres composés préférés encore sont ceux où m est zéro.

Les composés de formule (I) peuvent être préparés en traitant un composé de formule (II) : 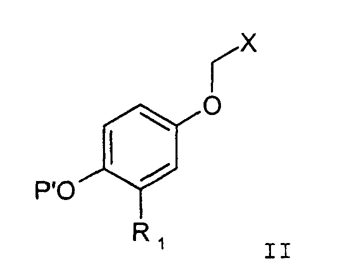 dans laquelle R₁ est tel qu'indiqué ci-dessus, P' est un groupe protecteur et X est un groupe de formule (a) ou (b) 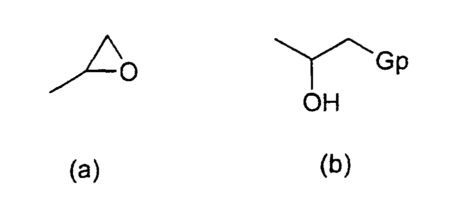 où Gp est un groupe partant tel que le tosylate, le mésylate ou un halogène,
avec une amine de formule (III) 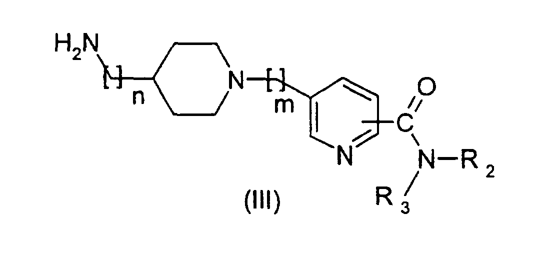 où n, m et CONR₂R₃ sont tels que définis ci-dessus, en clivant le groupe P' selon les méthodes usuelles et éventuellement transformant le composé de formule (I) ainsi obtenu en un de ses sels.

Plus particulièrement, la réaction entre les composés de formule (II) et (III) est réalisée dans un solvant organique, tel qu'un alcool inférieur comme le méthanol, l'éthanol et l'isopropanol ; le diméthylsulfoxyde ; un éther linéaire ou cyclique ; un amide comme le diméthylformamide ou le diméthylacétamide ; en utilisant des quantités au moins équimoléculaires des réactifs, éventuellement en faible excès d'amine.

La température de la réaction est comprise entre la température ambiante et la température de reflux du solvant choisi.

Comme groupes protecteurs P', on peut utiliser les groupes protecteurs usuels pour les groupes hydroxy tels que par exemple le méthoxyéthoxyméthyle (MEM) ou le benzyle.

Le clivage de ces groupes protecteurs est effectué selon les méthodes habituelles pour le groupe protecteur choisi ; dans le cas du groupe benzyle par exemple par hydrogénation en présence d'un catalyseur tel que le Pd/C dans un solvant convenable ; dans le cas du méthoxyéthoxyméthyle (MEM) on peut par contre utiliser un acide tel que l'acide trifluoroacétique.

La plupart des époxydes de formule (II) sont des composés connus en littérature ou bien ils peuvent être préparés par des procédés analogues à ceux décrits dans la littérature. Certains époxydes de formule (II) sont par exemple décrits dans WO 96/04233 et dans US 4,396,629. Selon ces méthodes, d'autres époxydes de formule (II) où R₁ est un groupe S(O)(C₁-C₄)Alk-R' peuvent être préparés à partir de phénols de formule (IV).

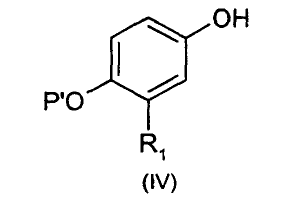

Les phénols de formule(IV) peuvent être préparées par déprotection sélective des composés de formule(V) où R₅ représente un groupe (C₂-C₄)Alk-R' et P' représente un groupe protecteur tel que le méthoxyéthoxyméthyle (MEM) ou le *para*-méthoxybenzyle (pMB). Le clivage de ces groupes protecteurs est effectué selon les méthodes habituelles connues de l'homme du métier pour le groupe protecteur choisi ; dans le cas du *para*-méthoxybenzyle (pMB) on peut utiliser un acide tel que l'acide trifluoroacétique.

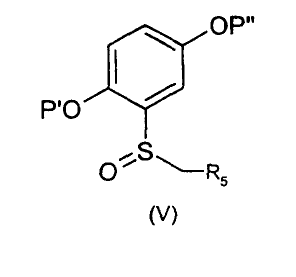

Les composés de formule (V) peuvent être préparés par alkylation de composés de formule (VI) au moyen d'une base forte, en présence d'un agent alkylant ; par exemple, au moyen de di-*iso*-propylamidure de lithium et d'iodure de méthyle dans le tétrahydofurane à -50°C.

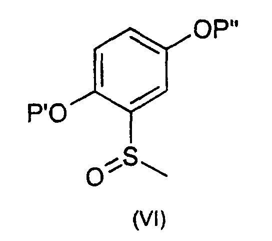

Les composés de formule(VI) peuvent être préparées par protection de la fonction phénol de composés de formule (VII), ou P'' représente un groupe protecteur tel que le méthoxyéthoxyméthyle (MEM) ou le *para*-méthoxybenzyle (pMB) ; par exemple, par action de chlorure de *para*-méthoxybenzyle en présence d'une base tel que l'hydrure de sodium, dans un solvant comme le diméthylformamide.

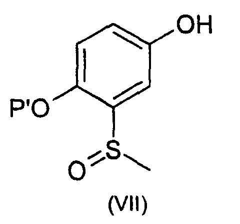

La plupart des phénols de formule (VII) sont des composés connus en littérature ou bien ils peuvent être préparés par des procédés analogues à ceux décrits dans la littérature. Certains phénols de formule (VII) sont par exemple décrits dans WO 96/04233 et dans US 4,396,629.

Les amines de formule (III) peuvent être préparées par réaction des pyridines convenables de formule (VIIIa) ou (VIIb)

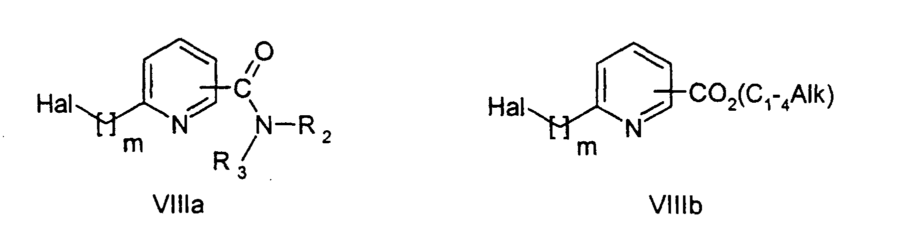

Où Hal représente un halogène et CONR₂R₃ et m sont tels que définis ci-dessus, avec une pipéridine de formule (IX) ci-dessous

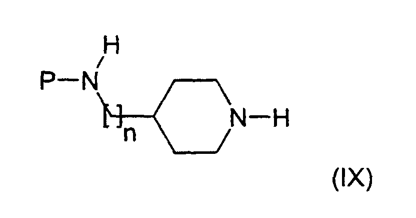

Où n est tel que défini ci-dessus et P représente un groupe protecteur, dans un solvant organique en présence d'une base, suivie par clivage du groupe P des composés de formule (X) ainsi obtenus.

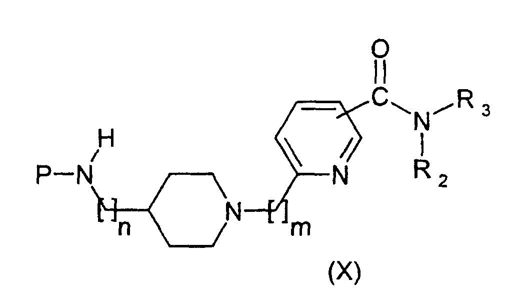

Le groupe amide CONR₂R₃ est introduit sur la pyridine par formation d'un lien amide entre un acide carboxylique et l'amine appropriée, éventuellement protégée sur les groupes fonctionnels pouvant interférer, en présence d'agent de couplage tel que 1-[3-(diméthylamino)propyl]-3-éthylcarbodiimide (DCI), dicyclohexylcarbodiimide(DCC),1-hydroxy-benzotriazole (HOBt), (benzotriazol-1-yloxy) tris-(diméthylamino)phosphonium hexafluoriphosphate (BOP).

Comme solvant de réaction, on peut bien utiliser par exemple le diméthylformamide, la pyridine, le diméthylsulfoxyde, un éther linéaire ou cyclique ou un solvant chloruré tel que le dichlorométhane.

Comme base on peut utiliser par exemple un hydroxyde alcalin, un carbonate alcalin tel que le carbonate de potassium ou une amine tertiaire telle que la triéthylamine.

La réaction de condensation ci-dessus est complétée en quelques heures, normalement en 2-12 heures.

La température de réaction est comprise entre la température ambiante et la température de reflux du solvant choisi.

Comme groupes protecteurs P, on peut utiliser les groupes protecteurs usuels pour les amines tels que par exemple le *tert*-butoxycarbonyle, l'acétyle, le carbobenzyloxy.

Le clivage de ces groupes protecteurs est effectué selon les méthodes habituelles décrites suivant le groupe protecteur choisi ; dans le cas du *tert*-butoxycarbonyle par exemple, le clivage est normalement effectué par hydrolyse acide.

Par ailleurs, les composés de formule (I) peuvent être préparés en traitant l'acide de formule (XI) : 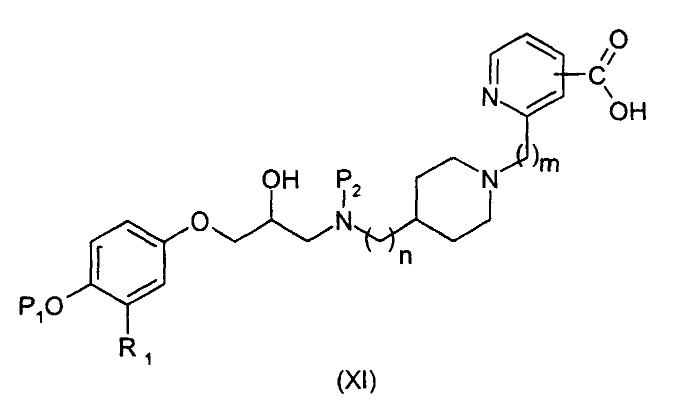 dans laquelle n, m et R₁ sont tel que définis ci-dessus, P₁ et P₂ sont des groupes protecteurs, avec une amine de formule (XII) en présence d'un agent de couplage, 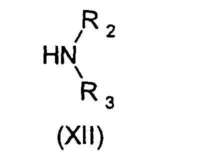 où R₂ et R₃ sont tels que définis ci-dessus, en clivant les groupes P₁ et P₂ selon les méthodes usuelles et éventuellement transformant le composé de formule (I) ainsi obtenu en un de ses sels.

Plus particulièrement, la réaction entre les composés de formule (XI) et (XII) est réalisée dans un solvant organique, tel que le diméthylformamide, la pyridine, le diméthylsulfoxyde, un éther linéaire ou cyclique ou un solvant chloruré tel que le dichlorométhane.

En présence d'un agent de couplage tel que DCI, DCC, EDCI, HOBt, BOP.

Comme base on peut utiliser par exemple un hydroxyde alcalin, un carbonate alcalin tel que le carbonate de potassium ou une amine tertiaire telle que la triéthylamine.

La formation de l'amide ci-dessus est complétée en quelques heures, normalement en 12-24 heures.

La température de réaction est comprise entre la température ambiante et la température de reflux du solvant choisi.

Comme groupes protecteurs P₁, on peut utiliser les groupes protecteurs usuels pour les groupes hydroxy tels que par exemple le méthoxyéthoxyméthyle (MEM) ou le benzyle.

Le clivage de ces groupes protecteurs est effectué selon les méthodes habituelles pour le groupe protecteur choisi ; dans le cas du groupe benzyle par exemple par hydrogénation en présence d'un catalyseur tel que le Pd/C dans un solvant convenable ; dans le cas du méthoxyéthoxyméthyle (MEM) on peut par contre utiliser un acide tel que l'acide trifluoroacétique.

Comme groupes protecteurs P₂, on peut utiliser les groupes protecteurs usuels pour les amines tels que par exemple le *tert*-butoxycarbonyle, l'acétyle, le carbobenzyloxy.

Le clivage de ces groupes protecteurs est effectué selon les méthodes habituelles décrites le groupe protecteur choisi ; dans le cas du *tert*-butoxycarbonyle par exemple, le clivage est normalement effectué par hydrolyse acide.

Les acides de formule(XI) peuvent être préparées par hydrolyse des esters(XIII)dans laquelle R₆ représente un groupe C₁₋₆ alkyl, selon des méthodes connues de l'homme du métier par exemple, par traitement par une base telle que la soude dans un mélange de solvant telle que le méthanol, l'eau et le tétrahydrofuranne.

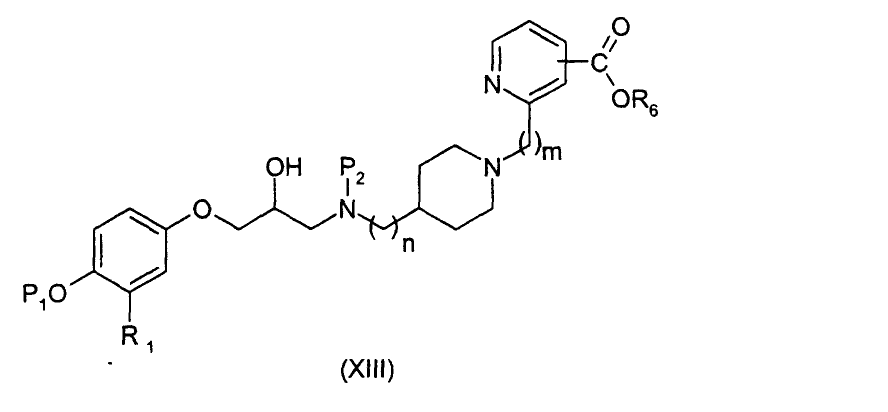

Les esters de formule(XIII) peuvent être préparées par protection de la fonction amine secondaire des esters(XIV)dans laquelle R₆ représente un groupe C₁₋₆ alkyl, selon des méthodes connues de l'homme du métier par exemple, par action du di-*tert*-butyl dicarbonate dans un solvant telle que l'acétate d'éthyle.

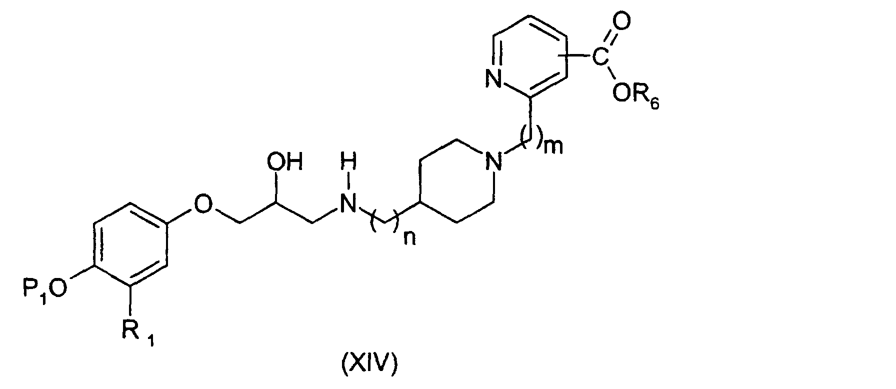

La préparation des esters de formule(XIV)est décrite dans WO 99/65895.

Les composés de formule (I) ont montré une affinité très puissante vis-à-vis des récepteurs β₃.

L'activité des composés de la présente invention vis-à-vis de l'activité β₃ a été mise en évidence à l'aide d'essais in vitro sur le colon humain selon la méthode décrite dans EP-B-436435 et dans T.Croci et al, Br. J. Pharmacol., 1997, 122 : 139P.

Plus particulièrement, on a constaté que les composés de formule (I) sont beaucoup plus actifs sur le côlon isolé que sur l'oreillette et sur la trachée.

Ces propriétés surprenantes des composés de formule (I) et permettent d'envisager leur utilisation comme médicaments à action β₃.

De plus, les composés de formule (I) sont peu toxiques ; notamment, leur toxicité aiguë est compatible avec leur utilisation comme médicaments pour le traitement de maladies dans lesquelles les composés ayant une affinité pour le récepteur β₃ trouvent leur application. Les composés de formule (I), ainsi que leurs sels pharmaceutiquement acceptables, peuvent donc être indiqués par exemple dans le traitement des maladies gastro-intestinales telles que le syndrôme du colon irritable, comme modulateurs de la motricité intestinale, comme lipolytiques, agents anti-obésité, anti-diabétiques, psychotropes, anti-glaucomateux, cicatrisants, anti-dépressants, comme inhibiteur des contractions utérines, comme tocolytiques pour prévenir ou retarder les accouchements précoces, pour le traitement et/ou la prophylaxie de la dysménorrhée.

L'utilisation des composés de formule (I) ci-dessus, ainsi que celle de leurs sels et solvates pharmaceutiquement acceptables pour la préparation de médicaments ci-dessus, constitue un aspect ultérieur de la présente invention.

Pour une telle utilisation, on administre aux mammifères qui nécessitent un tel traitement une quantité efficace d'un composé de formule (I) ou d'un de ses sels et solvates pharmaceutiquement acceptables.

Les composés de formule (I) ci-dessus et leurs sels et solvates pharmaceutiquement acceptables peuvent être utilisés à des doses journalières de 0,01 à 20 mg par kilo de poids corporel du mammifère à traiter, de préférence à des doses journalières de 0,1 à 10 mg/kg. Chez l'être humain, la dose peut varier de préférence de 0,5 mg à 1500 mg par jour, notamment de 2,5 à 500 mg selon l'âge du sujet à traiter, le type de traitement, prophylactique ou curatif, et la gravité de l'affection. Les composés de formule (I) sont généralement administrés en unité de dosage de 0,1 à 500 mg, de préférence de 0,5 à 100 mg de principe actif, une à cinq fois par jour.

Lesdites unités de dosage sont de préférence formulées dans des compositions pharmaceutiquement dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ci-dessus ou un de ses sels et solvates pharmaceutiquement acceptables.

Dans les compositions pharmaceutiques de la présente invention pour l'administration orale, sublinguale, sous-cutanée, intramusculaire, intraveineuse, topique, transdermique ou rectale, les ingrédients actifs de formule (I) ci-dessus, leurs sels et solvates pharmaceutiquement acceptables peuvent être administrés sous formes unitaires d'administration, en mélange avec des supports pharmaceutiques classiques, aux animaux et aux êtres humains, pour le traitement des affections susdites. Les formes unitaires d'administration appropriées comprennent les formes par voie orale telles que les comprimés, les gélules, les poudres, les granulés et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration locale, on mélange le principe actif dans un excipient pour la préparation de crèmes ou onguents ou on le dissout dans un véhicule pour l'administration intraoculaire, par exemple, sous forme de collyre.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Selon un autre de ses aspects, la présente invention concerne une méthode de traitement des pathologies qui sont améliorées par une action β₃-agoniste, qui comprend administrer un composé de formule (I) ou l'un de ses sels ou solvates pharmaceutiquement acceptables.

Les composés de formule (I), notamment les composés (I) marquées par un isotope, peuvent aussi être utilisés comme outils de laboratoire dans des essais biochimiques.

Les composés de formule (I) se lient au récepteur β₃-adrénergiques. On peut donc utiliser ces composés dans un essai ordinaire de liaison (« binding »), dans lequel on emploie un tissu organique où ce récepteur est particulièrement abondant, et on mesure la quantité de composé (I) déplacé par un composé test, pour évaluer l'affinité dudit composé vis-à-vis des sites de liaison de ce récepteur particulier.

Un autre objet spécifique de la présente invention est donc un réactif utilisable dans les essais biochimiques, qui comprend au moins un composé de formule (I) convenablement marqué.

Les exemples qui suivent illustrent mieux l'invention. Les spectres de masse (non indiqués) ainsi que les ions moléculaires [M+H]⁺ confirment la structure des composés.

### PREPARATION 1

### 4-tert-butoxycarbonylamino-pipéridine

On mélange à la température ambiante pendant 2 heures 25 g (0,13 mole) de 4-amino-1-benzylpipéridine, 36,2 ml (0,26 mole) de triéthylamine et 31,2 g (0,143 mole) de di-tert-butyl-dicarbonate dans 200 ml de diméthylformamide. On verse le mélange dans de l'eau, on extrait à l'acétate d'éthyle, on lave à l'eau et on cristallise le produit ainsi obtenu dans 200 ml d'éther isopropylique. On obtient 33 g de 1-benzyl-4-tert-butoxycarbonylamino-pipéridine qu'on hydrogène dans un mélange de 200 ml d'éthanol et 100 ml de tétrahydrofurane en présence de 3 g de Pd/C à 10 %. Après filtration du catalyseur, on isole le composé du titre. P.f. 157-160°C.

### PREPARATION 2

### 4-tert-butoxycarbonylamino-1-(5-aminocarbonylpyridi-2-yl)-pipéridine.

On chauffe à 80°C pendant 18 heures un mélange de 3 g (0,015 mole) du produit de la préparation 1, 1,5 g (0,015 mole) de triéthylamine et 2,34 g (0,015 mole) de 6-chloronicotinamide dans 60 ml de diméthylformamide. Après refroidissement on ajoute de l'eau et on filtre le produit. On obtient ainsi 2,8 g du composé du titre. P.f. 255°C déc.

### PREPARATION 3

### 4-amino-1-(5-aminocarbonylpyrid-2-yl)-pipéridine et son dichlorhydrate hémihydraté.

On mélange 1,84 g (0,0057 mole) de produit de l'exemple 1 et 50 ml d'acétate d'éthyle. On y ajoute 50 ml d'une solution 3N d'acide chlorhydrique dans de l'acétate d'éthyle sous agitation et on laisse agiter à la température ambiante pendant 10 heures. On filtre et on lave à l'acétone. On obtient ainsi 1,67 g du composé du titre.
P.f. 290°C déc.

### PREPARATION 4

### 4-tert-butoxycarbonylamino-1-(5-éthoxycarbonylpyrid-2-yl)-pipéridine.

On fait réagir un mélange comme décrit dans l'exemple 1 mais en utilisant l'ester éthylique de l'acide 6-chloronicotinique au lieu du 6-chloronicotinamide. Après refroidissement, on y ajoute de l'eau, on extrait l'acétate d'éthyle, on sèche la phase organique sur du sulfate de sodium et on évapore le solvant sous pression réduite. On obtient le composé du titre. P.f. 140-142°C.

### PREPARATION 5

### 4-amino-1-(5-éthoxycarbonylpyrid-2-yl)-pipéridine et son dichlorhydrate hydraté.

En opérant comme décrit dans l'exemple 2 mais en utilisant le produit de l'exemple 3 au lieu du produit de l'exemple 1, on obtient le composé du titre. P.f. 148-150°C.

### PREPARATION 6

### (2S)-4-benzyloxy-3-méthylsulfinyl-1-(2,3-époxypropoxy)-benzène.

On dissout sous agitation et atmosphère d'azote 0,71 g de NaH au 60% (0,0177 mole) dans 15 ml de diméthylformamide on ajoute lentement (pendant 30 minutes) 4,45 g (0,0169 mole) de 4-benzyloxy-3-méthylsulfinylphénol (préparé selon le procédé décrit dans US 4,396,629) dans 35 ml de diméthylformamide et après, 4,40 g (0,0169 mole) de S(+)glycildylnosilate dans 10 ml de diméthylformamide. Après l'introduction du dernier réactif le mélange est laissé réagir à température ambiante pendant 3 heures. On ajoute de l'eau, on extrait à l'acétate d'éthyle et on évapore le solvant. On purifie le brut de raction par flash-chromatographie en éluant par un mélange CH₂Cl₂ :MeOH = 97/3. [α]₃₆₅ₙₘ = - 14,3° ; [α]₄₃₆ₙₘ = - 3,9° (C=1% en CH₃OH, t=20°). On obtient le composé du titre.
Le produit ainsi obtenu est soumis à une analyse HPLC aux conditions suivantes :
- phase stationnaire chirale : CHIRALCEL OD-H
- phase mobile : hexane/éthanol = 80/20 (0.5 ml/min).
On observe deux pics et TR₁ = 20,780 min. et TR₂ = 23,900 min. correspondant aux diastéréoisomères ayant différente configuration à l'atome de soufre.

### PREPARATION 7

### 4-tert-butoxycarbonylamino-1-(5-hydroxycarbonylpyridi-2-yl) piperidine

Une solution de 1.88g de 4-*tert*-butoxycarbonylamino-1-(5-éthoxycarbonylpyridi-2-yl)piperidine, (5.4 mmol) dans un mélange de 80 mL de méthanol, 90 mL d'une solution aqueuse molaire de soude et 30 mL de tetrahydrofuranne est mis sous agitation pendant 26 heures, puis acidifiée jusqu'à pH=6 au moyen d'une solution aqueuse molaire d'acide chlorhydrique. La solution est ensuite basifiée jusqu'à pH=8 au moyen d'une solution saturée aqueuse de carbonate de sodium. Les solvants organiques sont-évaporés sous pression réduite et on acidifie la solution aqueuse jusqu'à pH=4 au moyen d'une solution aqueuse à 10% d'acide citrique. Le précipité formé est filtré, puis lavé au méthanol froid pour donner la 4-*tert*-butoxycarbonylamino-1-(5-hydroxycarbonylpyridi-2-yl)piperidine sous forme d'un solide beige (1.75 g, 100 %). [M+H⁺]=322.4

### PREPARATION 8

### 4-tert-butoxycarbonylamino-1-(5-piperidin-1-ylcarbonylpyridi-2-yl)piperidine

Une solution de 0.4 g de 4-*tert*-butoxycarbonylamino-1-(5-hydroxycarbonylpyridi-2-yl)piperidine (1.24 mmol), de 0.826 g de benzotriazol-1-yloxy-tris(diméthylamino)phosphonium hexa fluorophosphate (1.86 mmol), de 0.408 mL de pipéridine (3.72 mmol) et de 0.4 mL de triéthylamine dans 8ml de dichlorométhane est mise sous agitation pendant 4 heures. De l'eau est ajoutée et la phase aqueuse est extraite trois fois au dichlorométhane. Les phases organiques sont séchées sur sulfate de magnésium et concentrées sous pression réduite. La 4-*tert*-butoxycarbonylamino-1-(5-piperidin-1-ylcarbonylpyridi-2-yl)piperidine est obtenu sous forme d'une huile (0.475 g, 98 %) après purification sur gel de silice (éluant : dichlorométhane / méthanol 96/4). [M+H⁺]=389.3

### PREPARATION 9

### 4-amino-1-(5-piperidin-1-ylcarbonylpyridi-2-yl)piperidine

Une solution de 0.475 g de 4-*tert*-butoxycarbonyl amino-1-(5-piperidin-1-ylcarbonylpyridi-2-yl)piperidine (1.24 mmol) et de 5 mL d'acide trifluoroacétique dans 20 mL de dichlorométhane est mise sous agitation pendant 1 heure. Le milieu réactionnel est concentré sous pression réduite, puis repris au dichlorométhane. La solution est basifiée (pH=9) au moyen d'une solution aqueuse saturée de carbonate de sodium. La phase aqueuse est extraite cinq fois au dichlorométhane. Les phases organiques sont séchées sur sulfate de magnésium et concentrées sous pression réduite. La 4-amino-1-(5-piperidin-1-ylcarbonylpyridi-2-yl)piperidine est obtenu sous forme d'une huile (0.310 g, 88 %). [M+H⁺]=289.4

### PREPARATION 10

### 3-[1-(5-piperidin-1-ylcarbonylpyridi-2-yl)-4-piperidininyl amino]-1-[(4-benzyloxy-3-méthylsulfinyl)-phénoxy]-2-propanol

Une solution de 0.1 g de 4-benzyloxy-3-méthylsulfinyl-1-(2,3-époxypropoxy)-benzène (0.31 mmol) et de 0.173 g de 4-amino-1-(5-piperidin-1-ylcarbonylpyridi-2-yl)piperidine (0.62 mmol) dans 15 mL d'éthanol est porté au reflux pendant 17 heures, puis le milieu réactionnel est concentré sous pression réduite. Le 3-[1-(5-piperidin-1-ylcarbonylpyridi-2-yl)-4-piperidinylamino]-1-[(4-benzyloxy-3-méthylsulfinyl)-phénoxy]-2-propanol est obtenu sous forme d'une huile (0.140 g, 73 %) après purification sur gel de silice (éluant : dichlorométhane / méthanol 95/5). [M+H⁺]=607.5

### EXEMPLE 1

### 3-[1-(5-piperidin-1-ylcarbonylpyridi-2-yl)-4-piperidininyl amino]-1-[(4-hydroxy-3-méthylsulfinyl)-phénoxy]-2-propanol et son tritrifluoroacétate

Une solution de 0.14 g de 3-[1-(5-piperidin-1-ylcarbonylpyridi-2-yl)-4-piperidinylamino]-1-[(4-benzyloxy-3-méthylsulfinyl)-phénoxy]-2-propanol (0.31 mmol) dans 4 mL d'acide trifluoroacétique est chauffée pendant 6 heures à 65°C, puis le milieu réactionnel est concentré sous pression réduite. Le 3-[1-(5-piperidin-1-ylcarbonylpyridi-2-yl)-4-piperidinylamino]-1-[(4-hydroxy-3-méthylsulfinyl)-phénoxy]-2-propanol est obtenu sous forme de tritrifluroacétate (0.1 g, 50%) après purification sur HPLC/MS préparative et évaporation des solvants.
Appareils : Deux pompes Shimatzu LC8 couplées à un spectromètre de masse API 100 PE sciex. Un contrôleur SCL-10A. Un injecteur-collecteur de fractions Gilson 215.
Phase stationnaire : YMC-Guardpack ODS, 50x20mm, S-5µm, 120A
Phase mobile : Eluant A : H₂O/MeOH 95/5 + CF₃COOH 0.05%
Eluant B : H₂O/MeOH 5/95 + CF₃COOH 0.05%
Débit : 30mL/min

### Gradient d'élution :

| **t (en min)** | %A | %B |
|---|---|---|
| 0 | 90 | 10 |
| 3 | 90 | 10 |
| 11 | 10 | 90 |
| 13 | 10 | 90 |

TR = 6.05 min, [M+H⁺]=517.4.
Le produit purifié a été analysé par HPLC aux conditions suivantes.
Appareils :Deux pompes Shimatzu LC8 couplé à un detecteur UV SPD10-A et à un spectromètre de masse API 100 PE sciex . Un contrôleur SCL-10A. Un injecteur-collecteur de fractions Gilson 215.
Phase stationnaire : YMC-Pack FL-ODS, 50x4.6mm, S-5µm, 120A
Phase mobile : Eluant A : H₂O/MeOH 95/5 + CF₃COOH 0.05%
Eluant B : H₂O/MeOH 5/95 + CF₃COOH 0.05%
Débit : 3mL/min

### Gradient d'élution :

| **t (en min)** | %A | %B |
|---|---|---|
| 0 | 90 | 10 |
| 1 | 90 | 10 |
| 9 | 10 | 90 |
| 10 | 10 | 90 |

TR = 4.07 min, [M+H⁺]=517.4.

### EXEMPLE 2

### 3-[1-(5-benzylaminocarbonylpyridi-2-yl)-4-piperidininyl amino]-1-[(4-hydroxy-3-méthylsulfinyl)-phénoxy]-2-propanol et son tritrifluoroacétate

En opérant comme décrit dans l'exemple 1, mais en utilisant la 4-amino-1-(5-benzylaminocarbonylpyridi-2-yl)piperidine au lieu de la 4-amino-1-(5-piperidin-1-ylcarbonylpyridi-2-yl) piperidine dans la préparation 10 on obtient le composé du titre.
TR = 4.57 min, [M+H⁺]=539.4.

### EXEMPLE 3

### 3-[1-[5-(3-methoxyphenyl)aminocarbonylpyridi-2-yl]-4-piperidininyl amino]-1-[(4-hydroxy-3-méthylsulfinyl)-phénoxy]-2-propanol et son tritrifluoroacétate

En opérant comme décrit dans l'exemple 1, mais en utilisant la 4-amino-1-[5-(3-méthoxyphenyl)aminocarbonylpyridi-2-yl]piperidine au lieu de la 4-amino-1-(5-piperidin-1-ylcarbonylpyridi-2-yl) piperidine dans la préparation 10 on obtient le composé du titre.
TR = 4.93 min, [M+H⁺]=555.3.

### EXEMPLE 4

### 3-[1-[5-benzylaminocarbonylpyridi-2-yl]-4-piperidininylaminométhyl]-1-[(4-hydroxy-3-méthylsulfinyl)-phénoxy]-2-propanol et son tritrifluoroacétate

En opérant comme décrit dans l'exemple 2, mais en utilisant la 4-aminométhyl-1-benzylpipéridine au lieu de 4-amino-1-benzylpipéridine dans la préparation 1 on obtient le composé du titre. TR = 4.81 min, [M+H⁺]=553.2.

### EXEMPLE 5

### 3-[1-[5-piperidin-1-ylcarbonylpyridi-2-yl]-4-piperidininylaminométhyl]-1-[(4-hydroxy-3-méthylsulfinyl)-phénoxy]-2-propanol et son tritrifluoroacétate

En opérant comme décrit dans l'exemple 1, mais en utilisant la 4-aminométhyl-1-benzylpipéridine au lieu de 4-amino-1-benzylpipéridine dans la préparation 1 on obtient le composé du titre. TR = 4.16 min, [M+H⁺]=531.3

### EXEMPLE 6

### 3-[1-[5-(3-methoxyphenyl)aminocarbonylpyridi-2-yl]-4-piperidininylaminométhyl]-1-[(4-hydroxy-3-méthylsulfinyl)-phénoxy]-2-propanol et son tritrifluoroacétate

En opérant comme décrit dans l'exemple 3, mais en utilisant la 4-aminométhyl-1-benzylpipéridine au lieu de 4-amino-1-benzylpipéridine dans la préparation 1 on obtient le composé du titre. TR = 5.10 min, [M+H⁺]=569.4.

### PRÉPARATION 11

### 3-[1-[5-éthoxycarbonylpyrid-2-yl]-4-piperidininyl-tert-butoxycarbonylaminométhyl]-1-[(4-benzoxy-3-méthylsulfinyl)-phénoxy]-2-propanol

Une solution de 0.9 g de 3-[1-[5-éthoxycarbonylpyrid-2-yl]-4-piperidininylaminométhyl]-1-[(4-benzoxy-3-méthylsulfinyl)-phénoxy]-2-propanol (1.55 mmol) et de 0.37 g de di-*tert*-butyl dicarbonate (1.705 mmol) dans l'acétate d'éthyle (25mL) est portée au reflux pendant 24 heures. Après retour à température ambiante et addition d'eau, la phase aqueuse est extraite deux fois à l'acétate d'éthyle. Les phases organiques sont réunies, séchées sur sulfate de magnésium et les solvants sont évaporés sous pression réduite. Le 3-[1-[5-éthoxycarbonylpyridi-2-yl]-4-piperidininyl-*tert*-butoxycarbonylaminométhyl]-1-[(4-benzoxy-3-méthylsulfinyl)-phénoxy]-2-propanol est obtenu sous forme d'une poudre blanche (0.32 g, 30%) après purification sur gel de silice (25g SiO₂, éluant : heptane / acétate d'éthyle 1 / 2 ).
[M+H⁺] =682.5.

### PRÉPARATION 12

### 3-[1-[5-hydroxycarbonylpyridi-2-yl]-4-piperidininyl-tert-butoxycarbonylaminométhyl]-1-[(4-benzoxy-3-méthylsulfinyl)-phénoxy]-2-propanol.

Une solution de 5g de 3-[1-[5-éthoxycarbonylpyridi-2-yl]-4-piperidininyl-*tert*-butoxycarbonylaminométhyl]-1-[(4-benzoxy-3-méthylsulfinyl)-phénoxy]-2-propanol (7,34 mmol) dans un mélange de méthanol (200mL) de tétrahydrofurane (100mL) et d'une solution aqueuse de soude 1N (55mL, 4éq.) est chauffée pendant 18 heures à 50°C. Après retour à température ambiante, une solution 1N d'acide chlorhydrique est additionnée jusqu'à pH1, puis on ajout une solution saturée en hydrogénocarbonate de sodium jusqu'à pH8. Les solvants volatils sont évaporés sous pression réduite, puis deux extractions à l'acétate d'éthyle de la phase aqueuse sont réalisées. On ajoute à la phase aqueuse une solution d'acide citrique 10% jusqu'à pH6. Cette phase aqueuse est extraite deux fois à l'acétate d'éthyle puis on rassemble les dernières phases organiques et les sèche sur sulfate de magnésium. Après évaporation du solvant sous pression réduite on obtient le 3-[1-[5-hydroxycarbonylpyridi-2-yl]-4-piperidininyl-*tert*-butoxycarbonylaminométhyl]-1-[(4-benzoxy-3-méthylsulfinyl)-phénoxy]-2-propanol sous forme d'un solide blanc (4.36 g, 91%) . [M+H⁺]=654.6.

### EXEMPLE 7

### 3-[1-[5-(morphol-4-yl)carbonylpyridi-2-yl]-4-piperidininyl aminométhyl]-1-[(4-hydroxy-3-méthylsulfinyl)-phénoxy]-2-propanol et son tritrifluoroacétate

Une solution de 0.1g de 3-[1-[5-hydroxycarbonylpyridi-2-yl]-4-piperidininyl-*tert*-butoxycarbonylaminométhyl]-1-[(4-benzoxy-3-méthylsulfinyl)-phénoxy]-2-propanol (0.15 mmol) de 0.04g de 1-hydroxy benzotriazole (0.3 mmol) de 0.06g de 1-éthyl-3-(3-diméthylamino-propyl) carbodiimide (0.3 mmol) de 0.45 mL de triéthylamine et de 0.04 mL de morpholine (0.45 mmol) dans 10mL de dichlorométhane est mise sous agitation pendant 21 heures. De l'eau est ajoutée et la phase organique est lavée trois fois à l'eau. Les phases organiques sont séchées sur sulfate de magnésium et concentrées sous pression réduite. Le brut réactionnel dans 5 mL d'acide trifluoroacétique est ensuite chauffée pendant 7 heures à 60°C, puis le milieu réactionnel est concentré sous pression réduite. Le 3-[1-[5-(morphol-4-yl) carbonylpyridi-2-yl]-4-piperidininylaminométhyl]-1-[(4-hydroxy-3-méthylsulfinyl)-phénoxy]-2-propanol est obtenu sous forme de tritrifluroacétate (0.20 g, 17%) après purification sur HPLC/MS préparative et évaporation des solvants Appareils : Deux pompes Shimatzu LC8 couplées à un spectromètre de masse API 100 PE sciex. Un contrôleur SCL-10A. Un injecteur-collecteur de fractions Gilson 215.
Phase stationnaire : YMC-Guardpack ODS, 50x20mm, S-5µm, 120A
Phase mobile : Eluant A : H₂O/MeOH 95/5 + CF₃COOH 0.05%
Eluant B : H₂O/MeOH 5/95 + CF₃COOH 0.05%
Débit : 30mL/min

### Gradient d'élution :

| **t (en min)** | %A | %B |
|---|---|---|
| 0 | 90 | 10 |
| 3 | 90 | 10 |
| 11 | 10 | 90 |
| 13 | 10 | 90 |

TR = 4.81 min, [M+H⁺]=533.7.
Le produit purifié a été analysé par HPLC aux conditions suivantes.
Appareils :Deux pompes Shimatzu LC8 couplé à un detecteur UV SPD10-A et à un spectromètre de masse API 100 PE sciex . Un contrôleur SCL-10A. Un injecteur-collecteur de fractions Gilson 215.
Phase stationnaire : YMC-Pack FL-ODS, 50x4.6mm, S-5µm, 120A
Phase mobile : Eluant A : H₂O/MeOH 95/5 + CF₃COOH 0.05%
Eluant B : H₂O/MeOH 5/95 + CF₃COOH 0.05%
Débit : 3mL/min

### Gradient d'élution :

| **t (en min)** | %A | %B |
|---|---|---|
| 0 | 90 | 10 |
| 1 | 90 | 10 |
| 9 | 10 | 90 |
| 10 | 10 | 90 |

TR = 3.40 min, [M+H⁺]=533.7.

### EXEMPLE 8

### 3-[1-[5-(2-methoxybenzyl)aminocarbonylpyridi-2-yl]-4-piperidininylaminométhyl]-1-[(4-hydroxy-3-méthylsulfinyl)-phénoxy]-2-propanol et son tritrifluoroacétate

En opérant comme décrit dans l'exemple 4, mais en utilisant la 2-methoxybenzylamine au lieu de la morpholine on obtient le composé du titre.
TR = 4.87 min, [M+H⁺]=583.5.

### PRÉPARATION 13

### 4-benzyloxy-3-méthylsulfinyl-phénol, p-méthoxybenzoate (pmb)

Une solution de 1 g de 4-benzyloxy-3-méthylsulfinyl-phénol (3.85 mmol) de 0.185 g d'hydrure de sodium à 60% dans l'huile (4.6 mmol) et de 0.678 mL de 4-méthoxychlorobenzyle (5 mmol)dans 20 mL de diméthylformamide est mis sous agitation pendant 2 heures. De l'eau est ajoutée, la phase aqueuse est extraite deux fois à l'acétate d'éthyle. Les phases organiques sont réunies, séchées sur sulfate de magnésium et les solvants sont évaporés sous pression réduite. Le 4-benzyloxy-3-éthylsulfinyl-phénol, pmb est obtenu sous forme d'un solide blanc (1.6 g, 88%) après trituration dans du pentane, filtration et séchage sous vide. [M+H⁺]=383.3.

### PRÉPARATION 14

### 4-benzyloxy-3-éthylsulfinyl-phénol, pmb

Une solution de 0.4 g de 4-benzyloxy-3-méthylsulfinyl-phénol pmb (1.05 mmol) dans 40 mL de tetrahydrofurane est refroidit à -50°C, puis 0.784 mL d'une solution dimolaire de di-*iso*-propylamidure de lithium (1.57 mmol) dans le tetrahydrofurane est ajoutée. Le mélange réactionnel est laissé sous agitation pendant 15 minutes, et 0.13 ml d'iodure de méthyle (1.1 mmol) est ajouté. Après 15 minutes d'agitation, du méthanol est ajoutée et les solvants sont évaporés sous pression réduite. Le 4-benzyloxy-3-éthylsulfinyl-phénol, pmb est obtenu sous forme d'un solide blanc (0.3 g, 72%) après purification sur gel de silice (40g SiO₂ , éluant : heptane / acétate d'éthyle 4 / 1 ).

### PRÉPARATION 15

### 4-benzyloxy-3-éthylsulfinyl-phénol

Une solution de 0.3 g de 4-benzyloxy-3-éthylsulfinyl-phénol, pmb ( 0.75 mmol) dans d'un mélange de 5mL d'acide trifluoroacétique et de 5mL de dichlorométhane est mis sous agitation à 0°C pendant 2 heures. Les solvants sont évaporés sous pression réduite. Le 4-benzyloxy-3-éthylsulfinyl-phénol (0.2 g, 95%) est obtenu sous forme d'un solide blanc après purification sur gel de silice (10g SiO₂ , éluant : heptane / acétate d'éthyle 1 / 1 ). [M+H⁺]=277.4.

### EXEMPLE 9

### 3-[1-[5-piperidin-1-ylcarbonylpyridi-2-yl]-4-piperidininylamino]-1-[(4-hydroxy-3-éthylsulfinyl)-phénoxy]-2-propanol et son tritrifluoroacétate

En opérant comme décrit dans l'exemple 1, mais en utilisant le 4-benzyloxy-3-éthylsulfinyl-phénol au lieu du 4-benzyloxy-3-méthylsulfinyl-phénol dans la préparation 6 on obtient le composé du titre. TR = 4.47 mn, [M+H⁺]=531.3

Le tableau qui suit illustre les structures chimiques et les propriétés chimiques de certains composés des exemples de formule (I) selon l'invention.

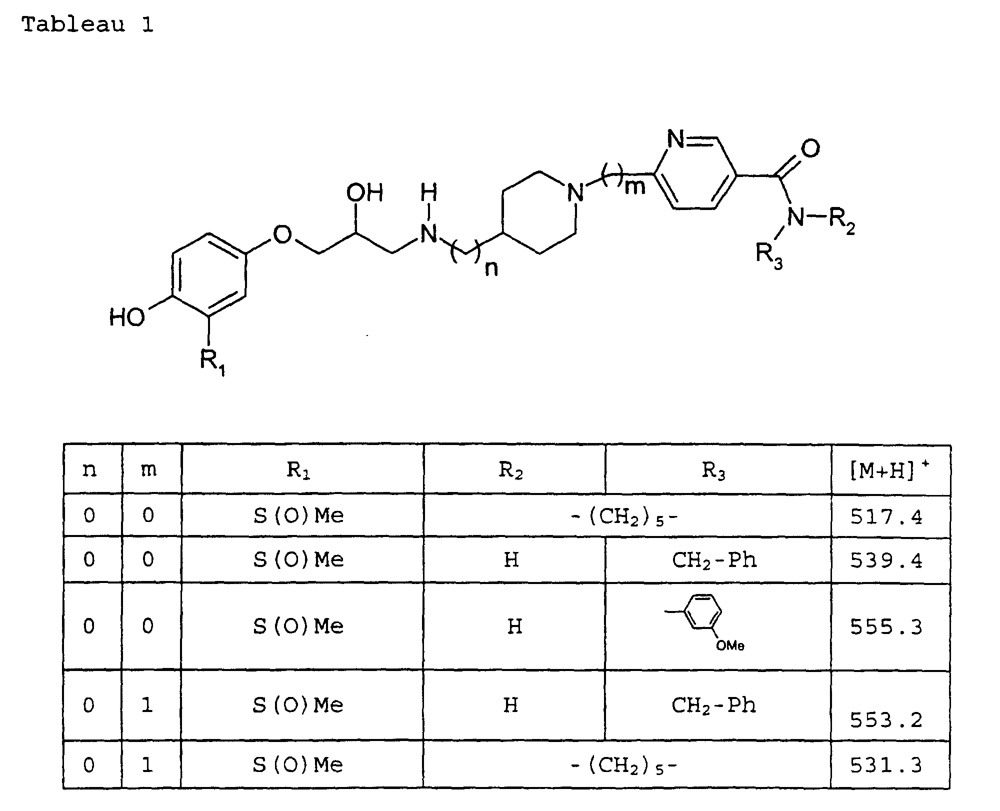

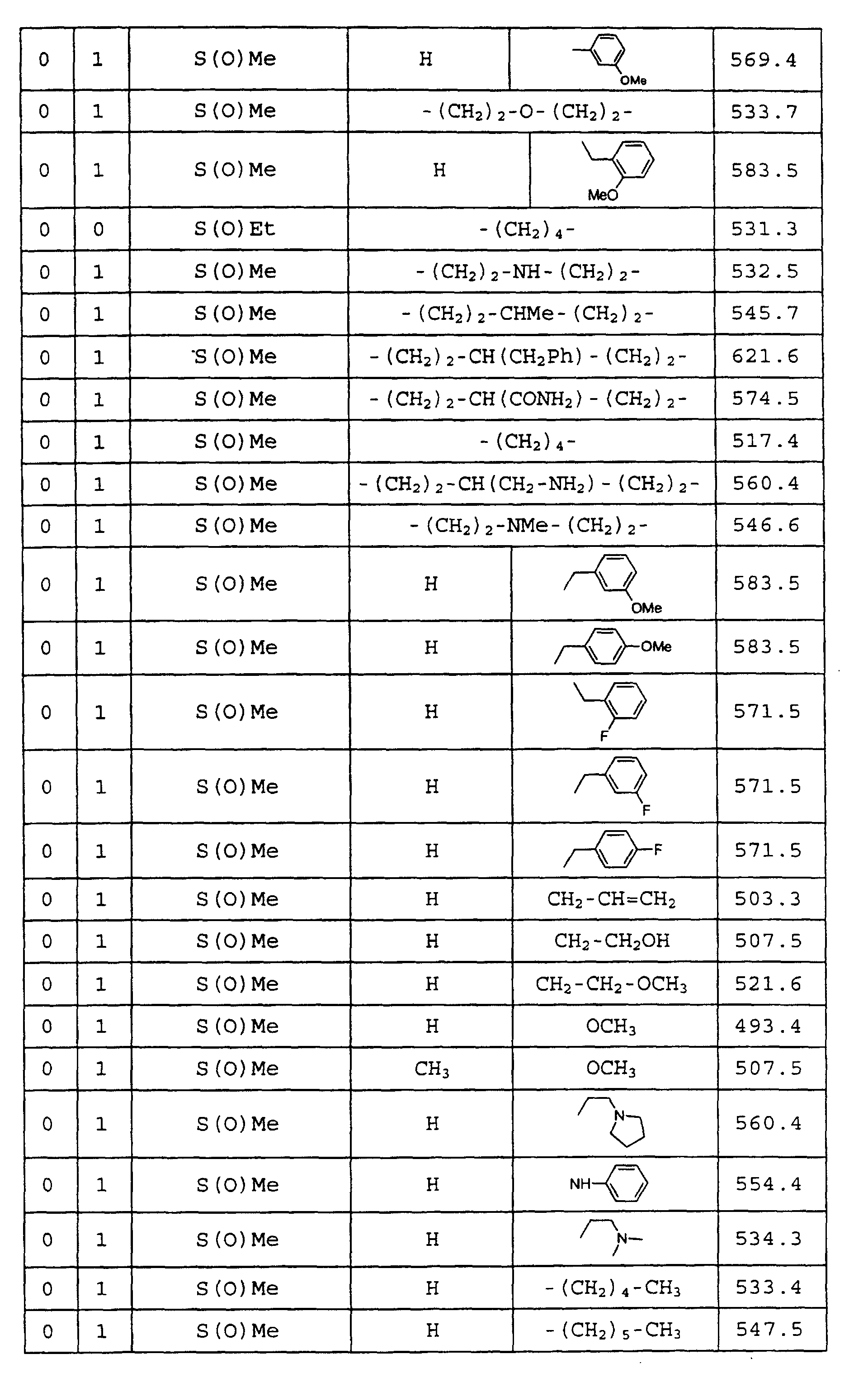

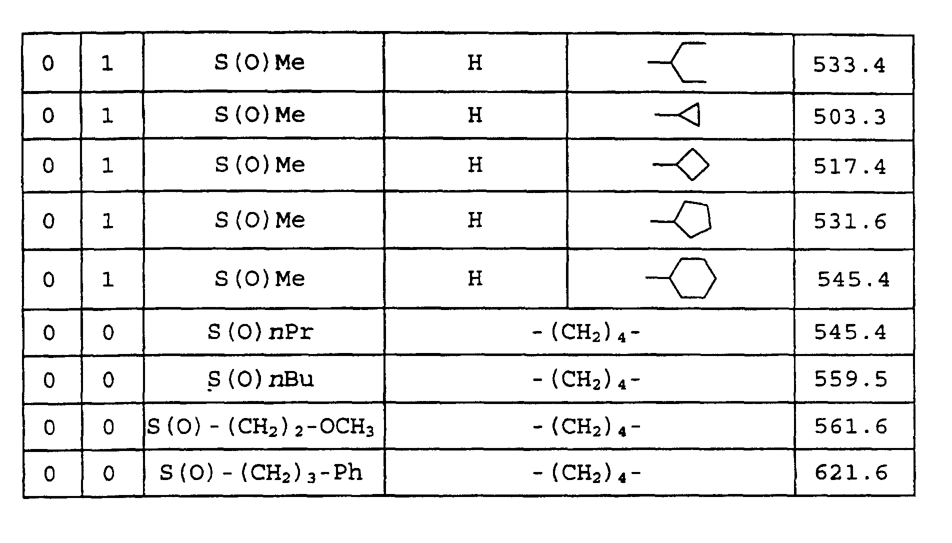

## Revendications

1. Phénoxypropanolamines de formule (I) 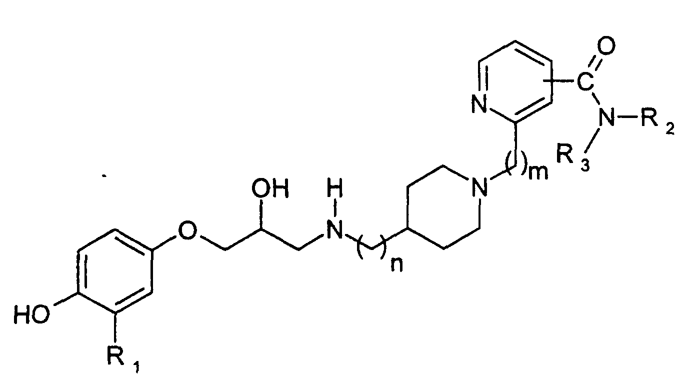 dans laquelle
R₁ représente un hydrogène, un groupe -S(O)_{z}-(C₁-C₄)Alk-R',
(R' étant H, phényle ou (C₁-C₄)alcoxy),-NHSO₂-(C₁-C₄)Alk, NHCO(C₁-C₄)Alk ;
m et n sont chacun indépendamment 0, 1 ou 2 ;
R₂ et R₃ représentent indépendamment un hydrogène, un groupe (C₅-C₆)alkyle, (C₃-C₆)cycloalkyle, (C₁-C₄)alcoxy, hydroxy(C₁-C₄)alkyle, (C₁-C₄)alcoxy-(C₁-C₄)alkyle, mono- ou di-(C₁-C₄)Alkamino(C₁-C₄)alkyle, pyrrolidino(C₁-C₄) alkyle, phénylamino, (C₂-C₄)alkenyle, un groupe aromatique ou hétéroaromatique substitué ou non par un groupe R₄, un groupe aralkyle ou hétéroaralkyle substitué ou non par un groupe R₄ ;R₂ et R₃ ne pouvant représenter simultanément l'hydrogène ;
R₂ et R₃ peuvent aussi ensemble constituer un cycle saturé ou insaturé de 3 à 8 atomes pouvant porter un substituant (C₁-C₄)alkyle, amino(C₁-C₄)alkyle, carbamoyle ou benzyle, ;
R₄ représente un hydrogène ou un halogène, un groupe -CO(C₁-C₄)Alk ou un groupe -NHSO₂-(C₁-C₄)Alk ; un groupe (C₁-C₄)Alk, un groupe (C₁-C₄)alkoxy, un halogène, -COOH, -COO(C₁-C₄)Alk, - CN, -CONR₃R₄, -NO₂, -SO₂NH₂, -NHSO₂(C₁-C₄)Alk ;
z est 1 ou 2 ;
et leurs sels ou solvates.

2. Composés selon la revendication 1, de formule (I) où le groupe CONR₂R₃ est en position 5 de la pyridine.

3. Composés selon la revendication 1, de formule (I) où m est zéro.

4. Procédé de préparation des composés de formule (I) selon la revendication 1, **caractérisé en ce que** l'on fait réagir un composé de formule (II) : 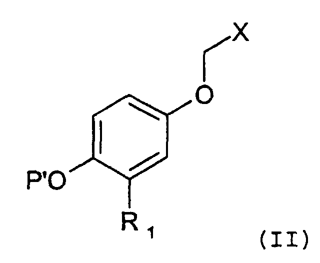 dans laquelle R₁ est tel que indiqué dans la formule (I), P' est un groupe protecteur et X est un groupe de formule (a) ou (b) 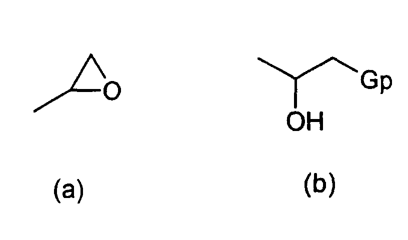 où Gp est un groupe partant tel que le tosylate, le mésylate ou un halogène,
avec une amine de formule (III) 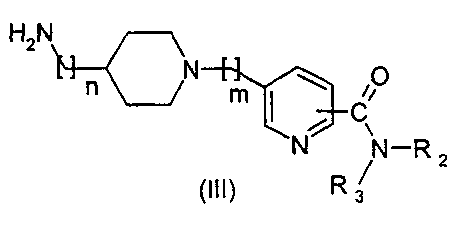 où n, m, R₂ et R₃ sont tels que définis dans la formule (I), en clivant le groupe P' selon les méthodes usuelles et éventuellement on transforme le composé de formule (I) ainsi obtenu en un de ses sels.

5. Composition pharmaceutique comprenant en tant que principe actif au moins un composé de formule (I) selon l'une des revendications 1 à 3, ou un de ses sels pharmaceutiquement acceptables.

6. Utilisation d'un composé selon l'une des revendications 1 à 3 pour la préparation de médicaments indiqués dans le syndrome du colon irritable, ou à action modulatrice de la mobilité intestinale, lipolytique, anti-obésité, anti-diabétique, psychotrope, anti-glaucomateuse, cicatrisante, antidépresseur, tocolytique.

## Patentansprüche

1. Phenoxypropanolamine der Formel (I) 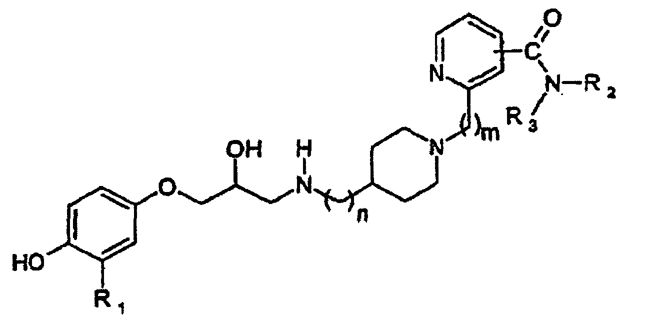 in der
R₁ Wasserstoff, eine Gruppe -S(O)_{z}-(C₁-C₄)-Alk-R' (worin R' H, Phenyl oder (C₁-C₄)-Alkoxy bedeutet), -NHSO₂-(C₁-C₄)-Alk oder NHCO-(C₁-C₄)-Alk darstellt;
m und n jeweils unabhängig voneinander 0, 1 oder 2 bedeuten;
R₂ und R₃ unabhängig voneinander Wasserstoff, eine (C₅-C₆)-Alkyl-, (C₃-C₆)-Cycloalkyl-, (C₁-C₄)-Alkoxy-, Hydroxy-(C₁-C₄)-alkyl-, (C₁-C₄)-Alkoxy-(C₁-C₄)-alkyl-, Mono- oder Di-(C₁-C₄)-alkamino-(C₁-C₄)-alkyl-, Pyrrolidino-(C₁-C₄)-alkyl-, Phenylamino-, (C₂-C₄)-Alkenyl-gruppe, eine gegebenenfalls durch eine Gruppe R₄ substituierte aromatische oder heteroaromatische Gruppe, eine gegebenenfalls durch eine Gruppe R₄ substituierte Aralkyl- oder Heteroaralkylgruppe bedeuten; wobei R₂ und R₃ nicht gleichzeitig Wasserstoff bedeuten;
wobei R₂ und R₃ auch gemeinsam einen gesättigten oder ungesättigten Ring mit 3 bis 8 Atomen bilden können, der einen (C₁-C₄)-Alkyl-, Amino-(C₁-C₄)-alkyl-, Carbamoyl- oder Benzyl-Substituenten tragen kann;
R₄ Wasserstoff oder ein Halogen, eine Gruppe -CO(C₁-C₄)-Alk oder eine Gruppe -NHSO₂-(C₁-C₄)-Alk; eine (C₁-C₄)-Alk-Gruppe, eine (C₁-C₄)-Alkoxygruppe, ein Halogen, -COOH, -COO(C₁-C₄)-Alk, -CN, -CONR₃R₄, -NO₂, -SO₂NH₂ oder -NHSO₂-(C₁-C₄)-Alk darstellt; und
z 1 oder 2 bedeutet;
und deren Salze oder Solvate.

2. Verbindungen nach Anspruch 1 der Formel (I), worin die Gruppe CONR₂R₃ in der 5-Stellung des Pyridins steht.

3. Verbindungen nach Anspruch 1 der Formel (I), in der m Null bedeutet.

4. Verfahren zur Herstellung der Verbindungen der Formel (I) nach Anspruch 1, **dadurch gekennzeichnet, daß** man eine Verbindung der Formel (II): 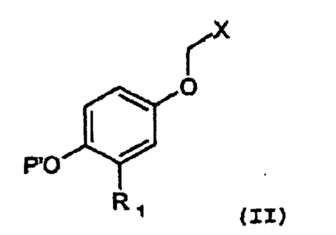 in der R₁ die bezüglich der Formel (I) angegebenen Bedeutungen besitzt, P' eine Schutzgruppe darstellt und X eine Gruppe der Formel (a) oder (b) bedeutet: 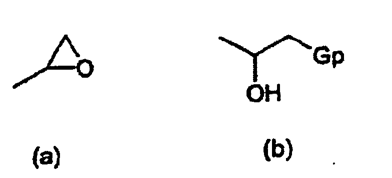 worin Gp eine austretende Gruppe, wie die Tosylat-, Mesylat-gruppe oder ein Halogenatom darstellt,
mit einem Amin der Formel (III) 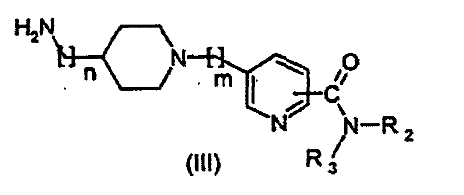 in der n, m, R₂ und R₃ die bezüglich der Formel (I) angegebenen Bedeutungen besitzen, unter Abspaltung der Gruppe P' gemäß an sich bekannter Verfahrensweisen umsetzt und gegebenenfalls die in dieser Weise erhaltene Verbindung der Formel (I) in eines ihrer Salze umwandelt.

5. Pharmazeutische Zubereitung enthaltend als Wirkstoff mindestens eine Verbindung der Formel (I) nach einem der Ansprüche 1 bis 3 oder eines ihrer pharmazeutisch annehmbaren Salze.

6. Verwendung einer Verbindung nach einem der Ansprüche 1 bis 3 für die Herstellung von Arzneimitteln, die für das Reizdarmsyndrom indiziert sind oder eine modulierende Wirkung auf die Darmmobilität, eine lipolytische, gegen Fettsucht gerichtete, antidiabetische, psychotrope, Antiglaukom-Wirkung, vernarbende Wirkung, antidepressive Wirkung oder tocolytische Wirkung besitzen.

## Claims

1. Phenoxypropanolamines of formula (I) 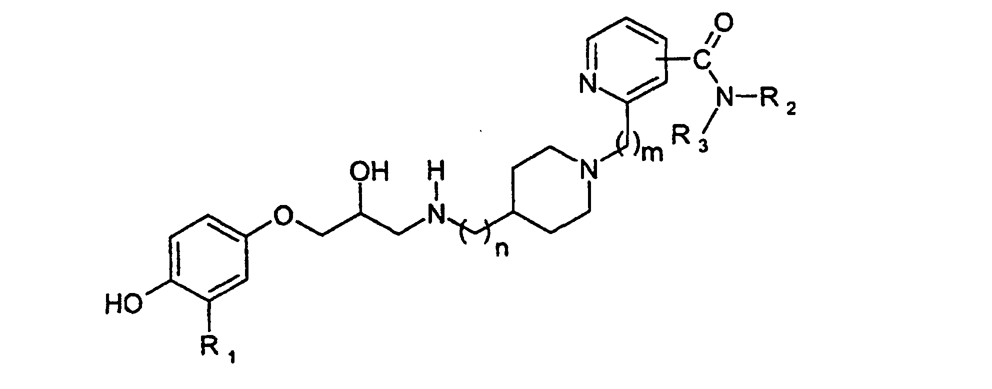 in which
R₁ represents a hydrogen, a group -S(O)_{z}-(C₁-C₄)alk-R', (R' being H, phenyl or (C₁-C₄)alkoxy), -NHSO₂-(C₁-C₄)alk or NHCO(C₁-C₄)alk;
m and n are each independently 0, 1 or 2;
R₂ and R₃ independently represent a hydrogen, a (C₅-C₆)alkyl, (C₃-C₆)cycloalkyl, (C₁-C₄)alkoxy, hydroxy(C₁-C₄)alkyl, (C₁-C₄)alkoxy-(C₁-C₄)alkyl, mono- or di(C₁-C₄)alkamino(C₁-C₄)alkyl, pyrrolidino (C₁-C₄)alkyl, phenylamino or (C₂-C₄)alkenyl group, an aromatic or heteroaromatic group optionally substituted with a group R₄, an aralkyl or heteroaralkyl group optionally substituted with a group R₄; R₂ and R₃ not simultaneously representing hydrogen;
R₂ and R₃ can also together constitute a saturated or unsaturated ring of 3 to 8 atoms possibly bearing a (C₁-C₄)alkyl, amino(C₁-C₄)alkyl, carbamoyl or benzyl substituent;
R₄ represents a hydrogen or a halogen, a group -CO(C₁-C₄)alk or a group -NHSO₂-(C₁-C₄)alk; a group (C₁-C₄)alk, a group (C₁-C₄)alkoxy, a halogen, -COOH, -COO(C₁-C₄)alk, -CN, -CONR₃R₄, -NO₂, -SO₂NH₂ or -NHSO₂(C₁-C₄)alk;
z is 1 or 2;
and the salts or solvates thereof.

2. Compounds according to Claim 1, of formula (I) in which the group CONR₂R₃ is in position 5 on the pyridine.

3. Compounds according to Claim 1, of formula (I) in which m is zero.

4. Process for preparing the compounds of formula (I) according to Claim 1, **characterized in that** a compound of formula (II): 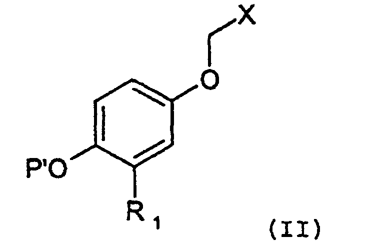 in which R₁ is as indicated in formula (I), P' is a protecting group and X is a group of formula (a) or (b) 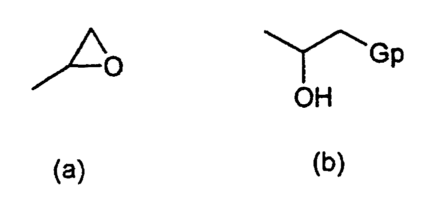 in which Gp is a leaving group such as tosylate, mesylate or a halogen,
is reacted with an amine of formula (III) 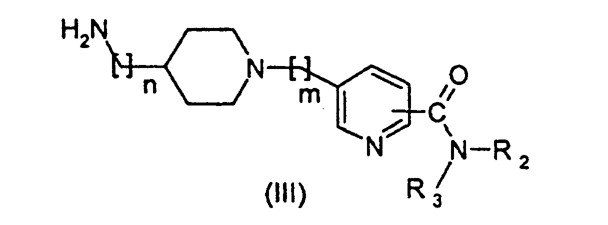 in which n, m, R₂ and R₃ are as defined in formula (I), by cleaving the group P' according to the usual methods, and optionally transforming the compound of formula (I) thus obtained into a salt thereof.

5. Pharmaceutical composition comprising, as active principle, at least one compound of formula (I) according to one of Claims 1 to 3, or a pharmaceutically acceptable salt thereof.

6. Use of a compound according to one of Claims 1 to 3, for the preparation of medicinal products indicated in irritable bowel syndrome, or with modulatory action on intestinal motility or lipolytic, anti-obesity, anti-diabetic, psychotropic, anti-glaucoma, cicatrizing, antidepressant or tocolytic action.
